# EUROPEAN PATENT APPLICATION

(11) **EP 4 231 036 A1**
(43) Date of publication of application: **23.08.2023**
(21) Application number: 22157815.6
(22) Date of filing: 21.02.2022
(51) Int. Cl.: G01R 33/56, A61B 5/055

(54) **DETECTION OF ARTIFICAL STRUCTURES IN MAGENTIC RESONANCE IMAGES DUE TO NEURAL NETWORKS**

(71) Applicant: Koninklijke Philips N.V., 5656 AG Eindhoven (NL)
(72) Inventor: SOMMER, Karsten, Eindhoven (NL); VAN DEN BRINK, Johan Samuel, Eindhoven (NL)
(74) Representative: Philips Intellectual Property & Standards

(57) **Abstract**

Disclosed herein is a medical system (100, 500) comprising a memory (110) storing machine executable instructions (120) and an image processing module (122). wherein the image processing module comprises an image processing neural network portion (306) and an artificial structure prediction portion (308), wherein the image processing module comprises an input (300) configured for receiving magnetic resonance data (124). The image processing neural network portion comprises a first output (302) configured for outputting a corrected magnetic resonance image (126) in response to receiving the magnetic resonance data at the input. The artificial structure prediction portion comprises a second output (304) configured to output artificial structure data (128) descriptive of a likelihood of artificial structures in the corrected magnetic resonance image. The medical system further comprises a computational system (104) Execution of the machine executable instructions causes the computational system to: receive (200) the magnetic resonance data; receive (202) the corrected magnetic resonance image at the first output and the artificial structure data at the second output in response to inputting the magnetic resonance data into the input of the image processing module; and provide (204) a warning signal (130) depending on the artificial structure data meeting a predetermined criterion.

## Description

### TECHNICAL FIELD OF THE INVENTION

The invention relates to magnetic resonance imaging, in particular to processing magnetic resonance images with neural networks.

### BACKGROUND OF THE INVENTION

In recent years neural networks have been used to either reconstruct, filter, or correct magnetic resonance images. An advantage of this approach is that the neural networks are very fast and they can be trained to perform complex image and reconstruction tasks. A disadvantage of using neural networks is that the neural network can add structures to the image that are not real. This is referred to in the literature as a hallucination. Herein artificial structure and hallucination are used as synonyms. The presence of an artificial structure or hallucination in a magnetic resonance image can have negative effects, because the physician inspecting the magnetic resonance image may be looking at anatomical features that are different from the actual subject.

United States patent application publication US 2020/0249300 discloses reconstructing magnetic resonance images from accelerated magnetic resonance imaging (MM) data. In one embodiment, a method for reconstructing a magnetic resonance (MR) image includes: estimating multiple sets of coil sensitivity maps from undersampled k-space data, the undersampled k-space data acquired by a multi-coil radio frequency (RF) receiver array; reconstructing multiple initial images using the undersampled k-space data and the estimated multiple sets of coil sensitivity maps; iteratively reconstructing, with a trained deep neural network, multiple images by using the initial images and the multiple sets of coil sensitivity maps to generate multiple final images, each of the multiple images corresponding to a different set of the multiple sets of sensitivity maps; and combining the multiple final images output from the trained deep neural network to generate the MR image.

### SUMMARY OF THE INVENTION

The invention provides for a medical system, a computer program product, and a method in the independent claims. Embodiments are given in the dependent claims.

Embodiments may provide a means of detecting artificial structures in magnetic resonance images by using an image processing module that comprises an image processing neural network that receives magnetic resonance data and outputs a corrected magnetic resonance image in response. In addition, the image processing module comprises an artificial structure detection portion that outputs artificial structure data in response to the magnetic resonance data being input into the image processing neural network. A variety of ways of implementing the image processing module are described below.

In one aspect the invention provides for a medical system that comprises a memory that stores machine-executable instructions and an image processing module. The image processing module may be machine-executable code or instructions also. The image processing module comprises an image processing neural network portion and an artificial structure prediction portion. The image processing module comprises input configured for receiving magnetic resonance data.

As used herein, magnetic resonance data encompasses either a magnetic resonance image or the k-space data that may be used to reconstruct a magnetic resonance image. As such, the image processing module is either configured to receive the k-space data or a magnetic resonance image as input. In the case where the image processing module receives the k-space data the image processing module reconstructs a magnetic resonance image from the k-space data. In the case where the magnetic resonance data is a magnetic resonance image, then the image processing module is used for performing some image processing task on this magnetic resonance image. This for example could be such things as noise removal, artifact removal, motion reduction or other such similar task.

The image processing neural network comprises a first output configured for outputting a corrected magnetic resonance image in response to receiving the magnetic resonance data at the input. The artificial structure prediction portion comprises a second output configured to output artificial structure data descriptive of a likelihood of artificial structures in the corrected magnetic resonance image. An artificial structure as used herein encompasses an anatomical structure or feature in the corrected magnetic resonance image that was artificially added by the image processing neural network portion. An artificial structure in the magnetic resonance image is also commonly known as a hallucination in the literature. The use of neural networks for constructing magnetic resonance images from k-space data or for performing various image processing tasks such as artifact or noise removal is well established. However, a common technical problem is that one cannot examine a neural network and understand what limitations it has and if it will provide data which is realistic or which has been altered and has so called hallucinations in it.

The incorporation of the artificial structure prediction portion provides a means of identifying possible artificial structures or hallucinations within the corrected magnetic resonance image. This may for example be useful when a physician or other medical professional is examining the corrected magnetic resonance image and would like to know what portions of the corrected magnetic resonance image may have errors or may be false. This can also be used as a control function. For example, if the image processing module is used to reconstruct an image from raw k-space data a different neural network or even an algorithmic method can be used to reconstruct the corrected magnetic resonance image instead thereby avoiding the possibility of artificial structures or hallucinations in the magnetic resonance image.

The medical system further comprises a computational system. Execution of the machine-executable instructions causes the computational system to receive the magnetic resonance data. Execution of the machine-executable instructions further causes the computational system to receive the corrected magnetic resonance image at the first output and the artificial structure data at the second output in response to inputting the magnetic resonance data into the input of the image processing module. Execution of the machine-executable instructions further causes the computational system to provide a warning signal depending on the artificial structure data meeting a predetermined criterion.

For example, the warning signal may be provided if the artificial data structure meets the predetermined criterion. The warning signal may take different forms in different examples. In one example the warning signal could be an audible or tactile warning. In other examples the warning signal could be a warning that is rendered by a display connected to the medical system. In yet other examples the warning signal could be a heat map or probability map indicating which locations within the corrected magnetic resonance image are likely to have artificial structures or hallucination structures.

The medical system may be incorporated into different types of systems. In one example the medical system may be a stand-alone system that processes k-space data into magnetic resonance images or processes magnetic resonance images. It may for example be located on a server or may be located remotely as a web service. In another example the machine-executable instructions and image processing module are incorporated into an algorithm or image processing module used to reconstruct magnetic resonance images. In yet other examples the medical system may incorporate or be incorporated into a medical imaging system such as a magnetic resonance imaging system.

In another embodiment the artificial structure data comprises template matching parameters for matching the corrected magnetic resonance image to a reference magnetic resonance image. Execution of the machine-executable instructions further causes the computational system to calculate an image difference map between the reference magnetic resonance image and the corrected magnetic resonance image using the template matching parameters. For example, the corrected magnetic resonance image may be morphed or transformed such that it matches the reference magnetic resonance image or vice versa. The image difference map may then look at individual pixels or voxels of the two images and calculate a difference between the two of them. In some examples the image difference map may be further processed.

For example, the image difference map may be thresholded so that only differences beyond a certain value or within a certain neighborhood are then registered. Calculating the image difference map may also involve some pre-processing of the corrected magnetic resonance image. For example, the contrast of the reference magnetic resonance image may be determined and the corrected magnetic resonance image may also be determined and then one or the other image is adjusted so that they both have the same contrast before the image difference map is calculated.

Execution of the machine-executable instructions further causes the computational system to determine if the image difference map meets the predetermined criterion algorithmically by determining if the image difference map exceeds a predetermined statistical measure. The warning signal is provided if the image difference map meets the predetermined criterion. As was mentioned previously, the predetermined statistical measure may be a thresholding process to see if there are voxels above a certain difference. Additional statistical criteria may also be applied. For example, the number of voxels which differs may also be a criteria or criterion used to determine if a warning signal is provided. The predetermined criterion may also involve looking at how clusters of voxels above a predetermined threshold are grouped together. For example, if there are maybe one or two isolated voxels this may not trigger a warning signal, but if there are regions above a certain size, which are also above the threshold, then this may be used in some examples to trigger the warning signal.

The template matching parameters could be different in different examples. For example, in one case it may be a three-dimensional deformation field that defines a per-pixel or voxel shift between the two images. The template matching parameters could also represent a lower dimensional shift based on identifying landmarks in both images, which then can be used to perform a transform process.

This embodiment may be beneficial because in many cases it is difficult to determine if there are hallucinations within a magnetic resonance image. Comparing the corrected magnetic resonance image to a reference magnetic resonance image may therefore provide a very effective and objective means of evaluating an image to see if there are artificial structures or hallucinations.

In another embodiment execution of the machine-executable instructions further causes the computational system to receive the subject metadata descriptive of the subject and choose the reference magnetic resonance image from a template database using the subject metadata. For example, the age, gender, and other details may be used to select a particular reference magnetic resonance image. This may have the advantage that the reference magnetic resonance image is closer to the anatomy of the subject.

In another embodiment the memory further comprises an image generation neural network configured to generation the reference magnetic resonance image in response to receiving the magnetic resonance data. For example, the artificial structure prediction portion and the image processing neural network portion are both implemented as neural networks. For example, an auto-encoder or an image generator trained using adversarial loss terms (as in the generative adversarial network concept) could be used to produce the reference magnetic resonance image.

In one example only the subject metadata is used as the input for the image generation neural network. In this case the image generation neural network could be trained using subjects with a normal anatomy.

In another embodiment the artificial structure prediction portion is implemented as a template matching algorithm. This embodiment may be beneficial because the image processing neural network portion and the artificial structure prediction portion can be two separate executable groups of code or programs. The artificial structure prediction portion may for example take the corrected magnetic resonance image as produced as its input and then determine the template matching parameters using an algorithm.

In another embodiment the artificial structure data comprises three-dimensional segmentation masks that define multiple pre-defined anatomical structures. For example, the artificial structure prediction portion may segment the corrected magnetic resonance image to determine the three-dimensional segmentation masks. This may be done using a conventional neural network or a segmentation algorithm such as a deformable shape algorithm. The machine-executable instructions further causes the computational system to receive predetermined volume ratio data descriptive of one or more ratios between the multiple pre-defined anatomical structures. Execution of the machine-executable instructions further causes the computational system to calculate measured volume ratio data descriptive of the one or more ratios between the multiple pre-defined anatomical structures from the artificial structure data. Execution of the machine-executable instructions further causes the computational system to determine if the predetermined criterion is met by comparing the predetermined volume ratio data and the measured volume ratio data.

In this embodiment the corrected magnetic resonance image may be segmented to determine these different three-dimensional segmentation masks. Then various ratios between these various volumes may be calculated and these ratios may be compared to the predetermined volume ratio data. If there is an artificial structure in the corrected magnetic resonance image or hallucination, then it is very likely that this will be detected by imbalance in the difference between the predetermined volume ratio data and the measured volume ratio data.

The segmentation can be performed in a variety of ways. It could for example be as a single neural network, which has a Y-net that could produce both the corrected magnetic resonance image and the artificial structure data. The image processing module could be a separate neural network and the artificial structure prediction portion could be a separate program or executable code also. For example, the artificial structure prediction portion could be a segmentation algorithm or it could be implemented as a separate neural network. This embodiment also has the advantage that the corrected magnetic resonance image is being compared to actual or live reference data for anatomical structures. This may be very effective in detecting artificial structures or hallucinations.

In another embodiment execution of the machine-executable instructions further causes the computational system to receive subject metadata. Execution of the machine-executable instructions further causes the computational system to select the predetermined volume ratio data from a volume ratio database using the subject metadata. This may be particularly effective because the various volume ratios can for example be fine-tuned into various parameters descriptive of the subject such as the age and gender.

In another embodiment the artificial structure prediction portion is implemented as an image segmentation algorithm. For example, it may be a conventional neural network which has been trained for performing image segmentations or it may for example be an algorithmic segmentation algorithm such as a deformable model.

In another embodiment the artificial structure prediction portion is implemented as a neural network. The artificial structure prediction portion is configured to receive the corrected magnetic resonance image as input. This is applicable both to the template matching embodiments as well as the embodiments that uses the three-dimensional segmentation masks. The neural network may for example be implemented as a ResNet neural network or a U-net neural network as several examples. The artificial structure prediction portion may be trained for example by taking a collection of magnetic resonance images and then either hand labeling the template matching parameters and the segmentations. This training data may then be used for example using a deep learning technique to train the artificial structure prediction portion if the artificial structure prediction portion is a convolutional neural network.

In another embodiment the artificial structure prediction portion is implemented as a neural network. The output artificial structure data is a spatially dependent probability map descriptive of the likelihood of artificial structures in the corrected magnetic resonance image. In this embodiment this spatially dependent probability map is output directly. There are several different ways to implement this. In one case, the artificial structure prediction portion and the image processing neural network portion could be implemented together as a single neural network, for example as the Y-net neural network which is described below. In another example, the artificial structure prediction portion and the image processing neural network portion are implemented as two separate neural networks. For example, these two neural networks may be two separate U-net neural networks.

In another embodiment the image processing neural network is a Y-net neural network. The Y-net neural network is formed from a U-net neural network structure configured for outputting a corrected magnetic resonance image at the first output in response to receiving the magnetic resonance data. The Y-net further comprises a decoding branch configured to output the artificial structure data that is descriptive of the artificial structures in the corrected magnetic resonance image at the second output in response to receiving the magnetic resonance data. The decoding branch is connected to the U-net neural network structure. The U-net neural network structure comprises the image processing neural network portion. The decoding branch comprises the artificial structure prediction portion.

As used herein a Y-net neural network encompasses a U-net neural network with an additional decoding branch. This embodiment may be beneficial because the addition of the additional decoding branch to the U-net may be an effective way of detecting hallucinations which are generated by the U-net neural network structure. The use of the Y-net neural network can be used for many of the embodiments described above. For example, the decoding branch can be used to output template matching parameters. In this case the U-net may be trained by using data which has a known input of magnetic resonance data, either an image or k-space data, and then the ground truth data may be the corrected magnetic resonance image and the appropriate template matching parameters.

The Y-net may also be useful for the embodiments which have the three-dimensional segmentation masks. In this case, the decoding branch performs a segmentation or predicts a segmentation of the corrected magnetic resonance image. In this case the training data may be known magnetic resonance data, again either in image or k-space, paired with ground truth data that has the corrected magnetic resonance image and reference segmentation masks and deep learning may be used for training the Y-net neural network. Likewise, the Y-net may be used for very effectively directly generating the spatially dependent probability map using the decoding branch. For example, this can be performed by taking magnetic resonance data, again in k-space or image space, and using the U-net portion to generate a corrected magnetic resonance image. Template data or a human could then go through and identify regions of the image which contain hallucinations, and this can be used as the ground truth data. The Y-net can then be trained again using deep learning.

In another embodiment the magnetic resonance data is descriptive of a brain of a subject. This embodiment may work particularly well because the structure or normal structure of neural anatomy is quite consistent from individual-to-individual. For example, the use of reference magnetic resonance images or the volume data may therefore be very accurate in predicting hallucinatory structures in the corrected magnetic resonance image.

In another embodiment the U-net neural network structure comprises a lowest resolution convolutional layer. The decoding branch is connected to the lowest resolution convolutional layer. This embodiment may be particularly beneficial because although the lowest resolution convolutional layer has a resolution that is typically lower than the corrected magnetic resonance image, it nonetheless contains data which is able to accurately describe where hallucinatory structures may be. In this case it may then be useful for predicting the template matching parameters, the volume ratios, or even outputting directly the spatially dependent probability map.

In another embodiment the magnetic resonance data is image data. In this case the magnetic resonance data is a magnetic resonance image that has already been reconstructed. The corrected magnetic resonance image is then a correction of the magnetic resonance data.

In another embodiment the image processing module is incorporated into a magnetic resonance imaging reconstruction algorithm configured to reconstruct a clinical magnetic resonance image in response to receiving k-space data. The magnetic resonance data is an intermediate magnetic resonance image calculated from the k-space data during the reconstruction of the clinical magnetic resonance image. This embodiment may be particularly beneficial because it may enable a means of providing more confidence when neural networks are incorporated into conventional magnetic resonance imaging reconstruction algorithms.

An example of this would be using the image processing module as a denoising filter used in a compressed sensing algorithm. In a compressed sensing algorithm, there is a denoising filter that is used before a data consistency step. The warning signal could then be used to retrigger data acquisition or it could be used to change the operation of the magnetic resonance imaging reconstruction algorithm. For example, if a large number of hallucinations are likely present, then the algorithm could switch to using a conventional algorithmic noise filter instead of the neural network-based noise filtering. This may help to increase the confidence that the clinical magnetic resonance image is more accurate and that it is less likely to contain unreal or hallucinatory artifacts.

In another embodiment the magnetic resonance data is k-space data. In this case the image processing module takes as input this k-space data and outputs a magnetic resonance image that has been corrected. The structure of this may take a variety of different forms. In one case the image processing neural network portion may perform the entire reconstruction and the artificial structure prediction portion could be completely separate. For example, the outputted image is used for template matching or has segmentation applied to it. In other cases, a Y-net structure could be trained to receive the k-space data and then output both the corrected magnetic resonance image and the artificial structure prediction portion.

In another embodiment the medical imaging system further comprises a magnetic resonance imaging system. The memory further comprises pulse sequence commands configured to control the magnetic resonance imaging system to acquire the magnetic resonance data such that it is descriptive of an imaging zone according to a magnetic resonance imaging protocol. In this example, acquiring the magnetic resonance data can have two meanings. In one case it may mean just acquiring the k-space data. This is the case when the image processing module reconstructs the corrected magnetic resonance image directly from the k-space data. The other interpretation that the magnetic resonance data may take is that the magnetic resonance data is the magnetic resonance image, in which the pulse sequence commands control the magnetic resonance imaging system to acquire k-space data which is then reconstructed into the magnetic resonance data by the computational system. Execution of the machine-executable instructions further causes the computational system to control the magnetic resonance imaging system to acquire the magnetic resonance data. As was mentioned before, this may also include an image reconstruction.

In another embodiment the image processing neural network portion is configured to perform noise removal.

In another embodiment the neural network portion is configured to perform artifact correction.

In another embodiment the neural network portion is configured to perform motion correction.

In another embodiment the neural network portion is configured to perform super-resolution.

In another embodiment the image processing neural network portion is configured to perform deblurring.

In another embodiment the image processing neural network portion is configured to perform a combination of the above-mentioned image processing techniques.

In another aspect the invention provides for a computer program product that comprises machine-executable instructions and an image processing module that may also be machine-executable instructions for execution by a computational system. The image processing module comprises an image processing neural network portion and an artificial structure prediction portion. The image processing module comprises an input configured for receiving magnetic resonance data. The image processing neural network portion comprises a first output configured to outputting a corrected magnetic resonance image in response to receiving the magnetic resonance data at the input. The artificial structure prediction portion comprises a second output configured to output artificial structure data descriptive of a likelihood of artificial structures in the corrected magnetic resonance image.

Execution of the machine-executable instructions causes the computational system to receive the magnetic resonance data. Execution of the machine-executable instructions further causes the computational system to receive the corrected magnetic resonance image at the first output and the artificial structure data at the second output both in response to inputting the magnetic resonance data into the input of the image processing module. Execution of the machine-executable instructions further causes the computational system to provide a warning signal depending on the artificial structure data meeting a predetermined criterion.

In another aspect the invention provides for a method of medical imaging. The method comprises receiving magnetic resonance data. Again, the magnetic resonance data may be either k-space data or it may be a magnetic resonance image depending upon the particular example. The method further comprises receiving the corrected magnetic resonance image at a first output of an image processing module and an artificial structure data at a second output of the image processing module in response to inputting the magnetic resonance data into an input of the image processing module. The artificial structure data is descriptive of a likelihood of artificial structures in the corrected magnetic resonance image. The image processing module comprises an image processing neural network portion and an artificial structure prediction portion. The image processing module comprises the input configured for receiving the magnetic resonance data. The image processing neural network comprises the first output configured for outputting the corrected magnetic resonance image in response to receiving the magnetic resonance data at the input. The artificial structure prediction portion comprises the second output configured to output the artificial structure data. The method further comprises providing a warning signal depending on the artificial structure data meeting a predetermined criterion.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan.

A Magnetic Resonance Imaging (MRI) image or MR image is defined herein as being the reconstructed two- or three-dimensional visualization of anatomic data contained within the k-space data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates an example image processing module;
Fig. 4 illustrates a further example of an image processing module;
Fig. 5 illustrates a further example of a medical system; and
Fig. 6 illustrates an example of how a template matching neural network can be trained.

### DETAILED DESCRIPTION OF EMBODIMENTS

Like numbered elements in these figures are either equivalent elements or perform the same function. Elements which have been discussed previously will not necessarily be discussed in later figures if the function is equivalent.

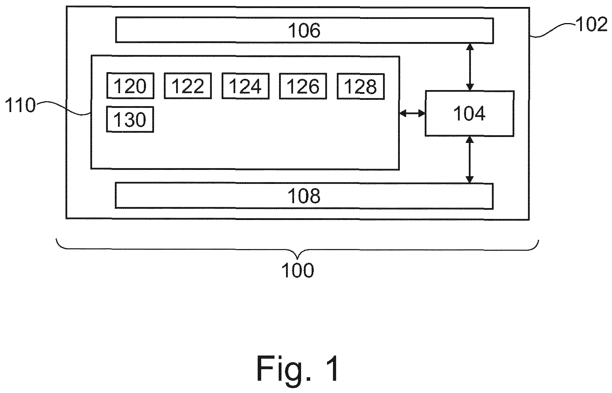

Fig. 1 illustrates an example of a medical system. In this example the medical system 100 is shown as comprising a computer 102 that has a computational system 104. The computational system 104 is shown as being in communication with an optional hardware interface 106 and an optional user interface 108. The optional hardware interface 106 enables the computational system 104 to communicate and exchange data with other components to control their operation and function. The user interface 108 may enable an operator or user to control the operation and function of the medical system 100.

The computational system 104 is shown as being further in communication with a memory 110. The memory 110 is intended to represent various types of memory and storage devices that may be in communication with the computational system 104. The memory 110 is shown as containing machine-executable instructions 120. The machine-executable instructions 120 enable the computational system 104 to perform various computational and control tasks. For example, the machine-executable instructions 120 may contain instructions which enable the computational system 104 to perform image processing or even reconstruct magnetic resonance images from k-space data. The memory 110 is further shown as containing an image processing module 122.

The image processing module has one input and two outputs. The input is configured for receiving magnetic resonance data which may be in various examples either k-space data or be a magnetic resonance image. The first output of the image processing module 122 is configured to output a corrected magnetic resonance image when the magnetic resonance data 124 is input. The second output of the image processing module 122 is configured to output artificial structure data. The artificial structure data is descriptive of a likelihood of artificial structures in the corrected magnetic resonance image. This could take different forms in different examples, for example it may be in some examples a heat diagram or plot, which shows the probability as a function of location within the corrected magnetic resonance image. In other cases, it may be a composite score for an entire corrected magnetic resonance image or for various portions of the corrected magnetic resonance image.

The memory 110 is further shown as containing magnetic resonance data 124. The memory is further shown as containing corrected magnetic resonance image 126 and artificial structure data 128 that were received from image processing module 122 in response to inputting the magnetic resonance data 124 into it. The memory 110 is further shown as containing a warning signal 130. The warning signal 130 may take different forms in different examples. For example, it may cause a display or interaction with the user interface 108 in some examples. In other examples the warning signal 130 may itself be a display or saving the value of various probabilities that the corrected magnetic resonance image 126 has artificial structures or hallucinations within it.

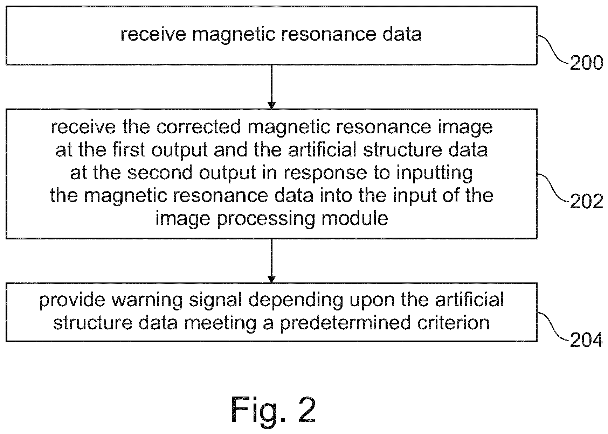

Fig. 2 shows a flowchart which illustrates a method of operating the medical system 100 of Fig. 1. First, in step 200, the magnetic resonance data 124 is received. Next, in step 202, the corrected magnetic resonance image 126 is received at the first output of the output and the artificial structure data 128 is received at the second output in response to inputting the magnetic resonance data 124 into the input of the image processing module 122. Finally, in step 204, the warning signal 130 is provided depending upon if the artificial data structure meets a predetermined criterion or not.

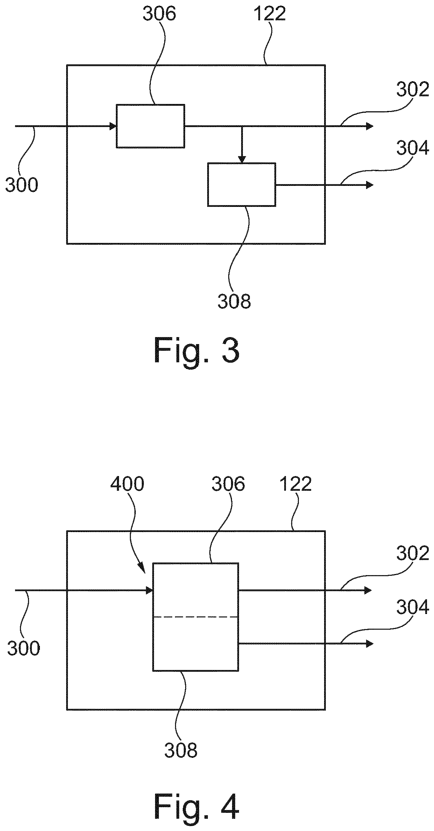

Fig. 3 illustrates one architecture of the image processing module 122. The image processing module is shown as having the input 300 and the first output 302 with the second output 304. Within the image processing module 122 the image processing neural network portion 306 and the artificial structure prediction portion 308 are separate. The image processing neural network portion 306 is an independent image processing neural network. It receives the magnetic resonance data 124 at its input and outputs the corrected magnetic resonance image 126.

The corrected magnetic resonance image 126 is then input directly into the artificial structure prediction portion 308. The artificial structure prediction portion 308 then outputs at the second output 304 the artificial structure data 128. The artificial structure prediction portion 308 may for example be implemented algorithmically or as a neural network. In one example the artificial structure prediction portion learns template matching between the corrected magnetic resonance image 126 and a reference magnetic resonance image. An image difference map can then be calculated between the reference magnetic resonance image and the corrected magnetic resonance image 126. This is beneficial because the artificial structures or hallucinatory structures within the corrected magnetic resonance image 126 can be detected using a means that is independent of a neural network.

In another example, the artificial structure prediction portion 308 comprises a segmentation algorithm. The segmentation algorithm segments the image processing module 122. Again, this may be implemented as a neural network that does the segmenting. However, this can be implemented algorithmically in a straightforward way using a variety of different algorithms such as a shape deformable model. These segmentations are equivalent to three-dimensional segmentation masks that define multiple predefined anatomical structures within the corrected magnetic resonance image 126. Various ratios can be calculated between these volumes and then compared to multiple predefined anatomical structure ratios. If the ratios from the measured and the reference data vary by more than a predetermined amount, this can be used to trigger the warning that there may be artificial or hallucinatory structures within the corrected magnetic resonance image. In some cases, this warning signal may be for the entire image or it may be localized where the various volumes that detected the inconsistency are located. In some cases, it may provide information on the location, in other cases it may only provide a warning for the overall corrected magnetic resonance image.

Fig. 4 illustrates a further structure of the image processing module 122. In this example there is a single neural network 400 that comprises the image processing neural network portion 306 and the artificial structure prediction portion 308. In this case, the artificial structure prediction portion 308 is a part of a neural network as is the image processing neural network portion 306. The neural network 400 may for example be a Y-net. In this case, the single neural network 400 may output the spatially dependent probability map directly at the second output 304.

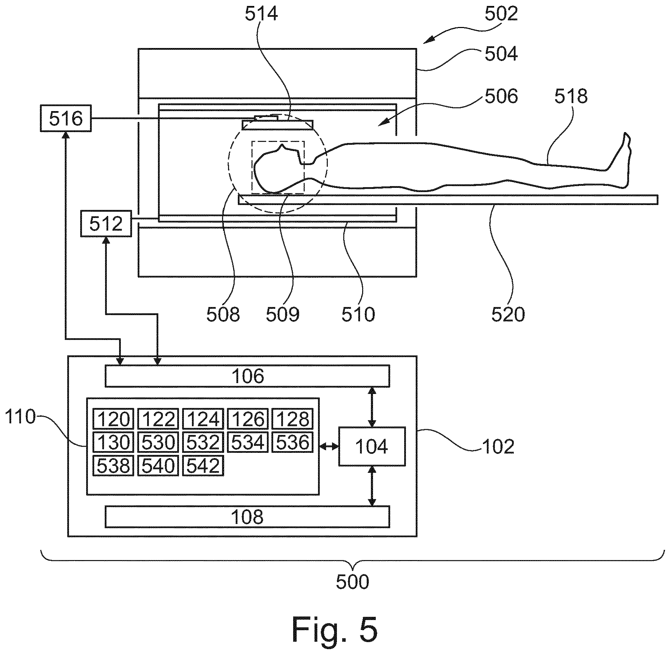

Fig. 5 illustrates a further example of a medical system 500. The example 500 illustrated in Fig. 5 is similar to the example 100 in Fig. 1 except that it additionally comprises a magnetic resonance imaging system that is controlled by the computational system 104.

The magnetic resonance imaging system 502 comprises a magnet 504. The magnet 504 is a superconducting cylindrical type magnet with a bore 506 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 506 of the cylindrical magnet 504 there is an imaging zone 508 where the magnetic field is strong and uniform enough to perform magnetic resonance imaging. A field of view 509 is shown within the imaging zone 508. The magnetic resonance data that is acquired typically acquried for the field of view 509. The region of interest could be identical with the field of view 509 or it could be a sub volume of the field of view 509. A subject 518 is shown as being supported by a subject support 520 such that at least a portion of the subject 518 is within the imaging zone 508 and the field of view 509.

Within the bore 506 of the magnet there is also a set of magnetic field gradient coils 510 which is used for acquisition of preliminary magnetic resonance data to spatially encode magnetic spins within the imaging zone 508 of the magnet 504. The magnetic field gradient coils 510 connected to a magnetic field gradient coil power supply 512. The magnetic field gradient coils 510 are intended to be representative. Typically magnetic field gradient coils 510 contain three separate sets of coils for spatially encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging zone 508 is a radio-frequency coil 514 for manipulating the orientations of magnetic spins within the imaging zone 508 and for receiving radio transmissions from spins also within the imaging zone 508. The radio frequency antenna may contain multiple coil elements. The radio frequency antenna may also be referred to as a channel or antenna. The radio-frequency coil 514 is connected to a radio frequency transceiver 516. The radio-frequency coil 514 and radio frequency transceiver 516 may be replaced by separate transmit and receive coils and a separate transmitter and receiver. It is understood that the radio-frequency coil 514 and the radio frequency transceiver 516 are representative. The radio-frequency coil 514 is intended to also represent a dedicated transmit antenna and a dedicated receive antenna. Likewise the transceiver 516 may also represent a separate transmitter and receivers. The radio-frequency coil 514 may also have multiple receive/transmit elements and the radio frequency transceiver 516 may have multiple receive/transmit channels. The transceiver 516 and the gradient controller 512 are shown as being connected to the hardware interface 106 of the computer system 102.

The memory 110 is further shown as containing pulse sequence commands 530 that may enable the computational system 104 to control the magnetic resonance imaging system 502 to acquire k-space data. In some examples the magnetic resonance data 124 is the k-space data. In other examples the magnetic resonance data 124 is reconstructed from the acquired k-space data. The memory 110 is further shown as containing an optional reference magnetic resonance image 532. The reference magnetic resonance image 532 may for example be from an anatomical atlas or may be a template image which is considered to represent normal anatomy of a subject.

In the case where the artificial structure prediction portion 308 determines template matching parameters the artificial structure data 128 may for example be a difference image between the reference magnetic resonance image 532 and the corrected magnetic resonance image 126 that was calculated using the template matching parameters to match the two images. The memory 110 is further shown as containing optional subject metadata 534. The optional subject metadata 534 may contain data which is descriptive of the subject such as the age, weight, and/or gender. This may be used to select the reference magnetic resonance image 532 from an optional template database 536.

As an alternative, the memory 110 is also shown as containing an optional image generation neural network 538. This for example could take the subject metadata 534 and have been trained to generate a reference magnetic resonance image 532. For example, the image generation neural network 538 may have been part of a GAN neural network that was trained.

The memory 110 is further shown as containing predetermined volume ratio data 540. This may be used by the artificial structure prediction portion 308 or the embodiment where the artificial structure data comprises three-dimensional segmentation masks that define multiple predefined anatomical structures. The memory 110 is also shown as containing an optional volume ratio database 542 that may for example be used to provide the predetermined volume ratio data 540 in response to providing the subject metadata 534.

As was mentioned above, neural-network-based correction of artifacts can lead to artificial structures (hallucinations) in the resulting images. These synthetic structures can have realistic appearance, which can complicate image interpretation and even lead to misdiagnosis.
The disclosed invention overcomes this problem by recasting the image correction setup, in some examples, into a multitask framework, which allows for automatic detection of network hallucinations. A dedicated multi-task network architecture may be used, with the primary task being the artifact correction. The secondary task is designed to allow for detection of artificial structures that may occur in the artifact-corrected images. Three examples of this framework are described in detail.

Image artifacts in are a common and persistent problem in the clinical application of magnetic resonance imaging (MRI). Since many artifact types (e.g., motion, Gibbs ringing, radial streaks) usually have a characteristic appearance, deep learning based post-processing methods have been shown to allow for substantial artifact reduction.

In certain cases, network-based image correction can lead to artificial structures ("hallucinations") in the resulting images. In contrast to 'classical' MR artifacts, which can usually be easily identified by an experienced operator, these synthetic structures can have realistic appearance. This may complicate image interpretation, and even lead to misdiagnosis if the hallucinations are not correctly identified.

Some examples may overcomes these problems by recasting the image correction setup into a multitask framework, which allows for automatic detection of network hallucinations.

A dedicated multi-task network architecture may be used in this framework, with the primary task being the artifact correction. The secondary task is designed to allow for detection of artificial structures that may occur in the artifact-corrected images.

In the following, three examples of this framework are described as illustrative examples.

### 1) Template matching

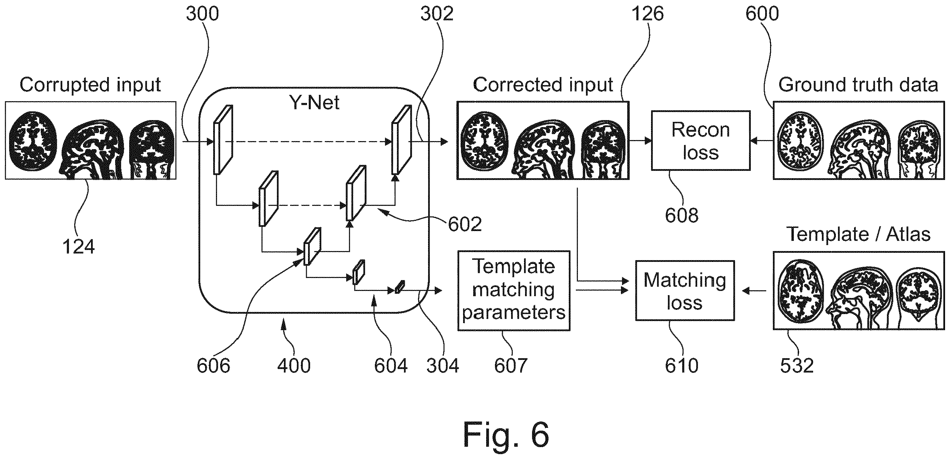

A schematic overview of this embodiment is shown in Fig. 6. Fig. 6 illustrates an example of how a template matching neural network can be trained. There is a single neural network 400 which is a Y-net. It has a U-net structure 602 and an additional decoding branch 604. In this example the decoding branch 604 has several additional convolutional layers. In other examples the decoding branch 604 could have a similar structure to the decoding branch of the U-net structure 602 complete with the additional skip connections. The decoding branch 604 is connected to the lowest resolution convolutional layer 606. The training data that is provided is a magnetic resonance data 124 paired with ground truth data 600 and a template or atlas 532. During training, the corrupted image 124 is input into the input 300. In response, the corrected image 126 is output by the first output 302. Also output at the second output 304 is the template matching parameters 607. The corrected output 126 is compared to ground truth data 600 using a reconstruction loss function 608. This reconstruction loss function 608 is used to provide loss data which is used to train the Y-net 400 starting at the first output 302. The corrected output 126, the template matching parameters 607, and the reference magnetic resonance image 532 are input into a matching loss function 610 which provides a matching loss which is used to train the decoding branch 604 starting at the second output 304.

For the example problem of motion artifact correction in MR brain scans, a tailored Y-Net architecture is used to simultaneously reduce artifacts as well as perform a matching of the artifact-corrected output to a reference brain atlas such as Talairach or MNI. To perform the template matching, the second decoding branch of the Y-Net is trained to provide template matching parameters that are required by the specific matching algorithm. In the most general case, these parameters are the 3D deformation fields that define a per-pixel shift for the entire volume. Assuming a certain degree of deformation field compressibility, a lower-dimensional representation can also be chosen to simplify the associated learning problem. During training, the weights of the Y-Net are optimized using a standard reconstruction loss function - such as mean-squared error - for the artifact correction branch and a matching loss function for the template matching branch. The latter is designed to achieve high sensitivity w.r.t. small anatomical differences, e.g. by using an edge-based loss function as proposed previously for super-resolution tasks.

During inference, potential hallucinations in the network-corrected images are detected by large deviations between the morphed artifact-corrected image and the template brain.

### 2) Volumetry

In another embodiment, the secondary decoding path of the Y-Net is trained to provide 3D segmentation masks for volumetric evaluation of pre-defined intracranial compartments and brain structures. In this implementation, reference masks are obtained by manual or automatic segmentation of the ground truth data, and the template matching loss is replaced e.g. by the cross-entropy loss function as commonly used for image segmentation tasks.
During inference, detection of potential hallucinations in the artifact-corrected data is realized by comparison of the volumetric results with standard reference values.

### 3) Direct prediction of hallucination risk

In another embodiment, the secondary decoding path of the Y-Net is trained to directly provide a hallucination risk, either as a global risk value or as a localized risk map. The latter can be used to guide image interpretation, highlighting "unreliable" image regions that are at high risk of containing artificial anatomical structures.

Other examples may also be considered:
- Patient or subject metadata is used as additional input data, thereby taking diversity of brain morphology into account. For the template matching embodiment, a dictionary of template brains with corresponding metadata is stored, and the appropriate dictionary entry is used during inference. For the volumetry embodiment, reference values are adjusted for the specific patient metadata.
- An additional dedicated network for hallucination detection is used, given the morphed artifact-corrected images and the template brain as input. This network is trained to ignore normal anatomical variations but detect atypical differences (hallucinations).
- Both Y-Net branches are trained separately: freeze weights of one branch while updating the other.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: image processing module
- 124: magnetic resonance data
- 126: corrected magnetic resonance image
- 128: artificial structure data
- 130: warning signal
- 300: input
- 302: first output
- 304: second output
- 306: image processing neural network portion
- 308: artificial structure prediction portion
- 400: single neural network
- 500: medical system
- 502: magnetic resonance imaging system
- 504: magnet
- 506: bore of magnet
- 508: imaging zone
- 509: field of view
- 510: magnetic field gradient coils
- 512: magnetic field gradient coil power supply
- 514: radio-frequency coil
- 516: transceiver
- 518: subject
- 520: subject support
- 530: pulse sequence commands (control commands)
- 532: reference magnetic resonance image
- 534: subject metadata
- 536: tempate database
- 538: image generation neural network
- 540: predetermiend volume ratio data
- 542: volume ratio database
- 600: ground truth data
- 602: U-net structure
- 604: decoding branch
- 606: lowest resolution convolutional layer
- 607: template matching parameters
- 608: reconstruction loss function
- 610: matching loss function

## Claims

1. A medical system (100, 500) comprising:
- a memory (110) storing machine executable instructions (120) and an image processing module (122), wherein the image processing module comprises an image processing neural network portion (306) and an artificial structure prediction portion (308), wherein the image processing module comprises an input (300) configured for receiving magnetic resonance data (124), wherein the image processing neural network portion comprises a first output (302) configured for outputting a corrected magnetic resonance image (126) in response to receiving the magnetic resonance data at the input, wherein the artificial structure prediction portion comprises a second output (304) configured to output artificial structure data (128) descriptive of a likelihood of artificial structures in the corrected magnetic resonance image,
- a computational system (104), wherein execution of the machine executable instructions causes the computational system to:
- receive (200) the magnetic resonance data;
- receive (202) the corrected magnetic resonance image at the first output and the artificial structure data at the second output in response to inputting the magnetic resonance data into the input of the image processing module; and
- provide (204) a warning signal (130) depending on the artificial structure data meeting a predetermined criterion.

2. The medical system of claim 1, wherein the artificial structure data comprises template matching parameters for matching the corrected magnetic resonance image to a reference magnetic resonance image, wherein execution of the machine executable instructions further causes the computational system to:
- calculate an image difference map between the reference magnetic resonance image template and the corrected magnetic resonance image using the template matching parameters; and
- determine if the image difference map meets the predetermined criterion algorithmically by determining if the image difference map exceeds a predetermined statistical measure, wherein the warning signal is provided if the image difference map meets the predetermined criterion.

3. The medical system of claim 2, wherein the artificial structure prediction portion is implemented as a template matching algorithm.

4. The medical system of claim 1, wherein the artificial structure data comprises three-dimensional segmentation masks that defines multiple pre-defined anatomical structures, wherein the machine executable instructions further causes the computational system to:
- receive predetermined volume ratio data (540) descriptive of one or more ratios between the multiple pre-defined anatomical structures;
- calculate measured volume ratio data descriptive of the one or more ratios between the multiple pre-defined anatomical structures from the artificial structure data; and
- determine if the predetermined criterion is met by comparing the predetermined volume ratio data and the measured volume ratio data.

5. The medical system of claim 4, wherein the artificial structure prediction portion is implemented as an image segmentation algorithm.

6. The medical system of any one of claims 1, 2, and, 4, wherein the artificial structure prediction portion is implemented as a neural network, wherein the artificial structure prediction portion is configured to receive the corrected magnetic resonance image as input.

7. The medical system of claim 1, wherein the artificial structure prediction portion is implemented as a neural network, wherein the output artificial structure data is a spatially dependent probability map descriptive of the likelihood of artificial structures in the corrected magnetic resonance image.

8. The medical system of any one of claims 1, 2, 3, 4, 5, and 7, wherein the image processing module is a Y-net neural network (400), wherein the Y-net neural network is formed from a U-net neural network structure (602) configured for outputting the corrected magnetic resonance image at the first output in response to receiving the magnetic resonance data at the input, wherein the Y-net further comprises a decoding branch (604) configured to output the artificial structure data descriptive of artificial structures in the corrected magnetic resonance image at the second output in response to receiving the magnetic resonance data, wherein the decoding branch is connected to the U-net neural network structure, wherein the U-net neural network comprises the image processing neural network portion, and wherein the decoding branch comprises the artificial structure prediction portion.

9. The medical system of any one of the preceding claims, wherein the magnetic resonance data is image data.

10. The medical system of claim 9, wherein the image processing module is incorporated into a magnetic resonance imaging reconstruction algorithm configured to reconstruct a clinical magnetic resonance image in response to receiving k-space data, wherein the magnetic resonance data is an intermediate magnetic resonance image calculated from the k-space data during the reconstruction of the clinical magnetic resonance image.

11. The medical system of any one of claims 1 through 7, wherein the magnetic resonance data is k-space data.

12. The medical system of any one of the preceding claims, wherein the medical imaging system further comprises a magnetic resonance imaging system (502), wherein the memory further comprises pulse sequence commands (530) configured to control the magnetic resonance imaging system to acquire the magnetic resonance data from an imaging zone according to a magnetic resonance imaging protocol, wherein execution of the machine executable instructions further causes the computational system to control the magnetic resonance imaging system to acquire the magnetic resonance data.

13. The medical system of any one of the preceding claims, wherein the image processing neural network portion is configured for any one of the following: noise removal, artifact correction, motion correction, de-blurring, and combinations thereof.

14. A computer program product comprising machine executable instructions (120) and an image processing module (122) for execution by a computational system (104), wherein the image processing module comprises an image processing neural network portion (306) and an artificial structure prediction portion (308), wherein the image processing module comprises an input (300) configured for receiving magnetic resonance data (124), wherein the image processing neural network portion comprises a first output (302) configured for outputting a corrected magnetic resonance image (126) in response to receiving the magnetic resonance data at the input, wherein the artificial structure prediction portion comprises a second output (304) configured to output artificial structure data (128) descriptive of a likelihood of artificial structures in the corrected magnetic resonance image, wherein execution of the machine executable instructions causes the computational system to:
- receive (200) the magnetic resonance data;
- receive (202) the corrected magnetic resonance image at the first output and the artificial structure data at the second output in response to inputting the magnetic resonance data into the input of the image processing module; and
- provide (204) a warning signal (130) depending on the artificial structure data meeting a predetermined criterion.

15. A method of medical imaging, wherein the method comprises:
- receiving (200) magnetic resonance data (124);
- receiving (202) a corrected magnetic resonance image (126) at a first output (302) of an image processing module (122) and artificial structure data (128) at a second output (304) of the image processing module in response to inputting the magnetic resonance data into an input (300) of the image processing module, wherein the artificial structure data is descriptive of a likelihood of artificial structures in the corrected magnetic resonance image, wherein the image processing module comprises an image processing neural network portion (306) and an artificial structure prediction portion (308), wherein the image processing module comprises the input configured for receiving the magnetic resonance data, wherein the image processing neural network portion comprises the first output configured for outputting the corrected magnetic resonance image in response to receiving the magnetic resonance data at the input, wherein the artificial structure prediction portion comprises the second output configured to output the artificial structure data; and
- providing (204) a warning signal (130) depending on the artificial structure data meeting a predetermined criterion.
